# FASCICULE DE BREVET EUROPEEN

(11) **EP 0 972 061 B1**
(45) Date de publication et mention de la délivrance du brevet: **06.09.2006**
(21) Numéro de dépôt: 98912557.0
(22) Date de dépôt: 26.02.1998
(51) Int. Cl.: C12N 15/82, C12N 15/12, C12N 1/21, A01H 5/00

(54) **UTILISATIONS DE LA STERILITE MALE CHEZ LES PLANTES**
ANWENDUNGSMOEGLICHKEITEN DER MAENNLICHEN STERILITAET VON PFLANZEN
USES OF MALE STERILITY IN PLANTS

(30) Priorité: 27.02.1997 FR 9702369
(43) Date de publication de la demande: 19.01.2000
(73) Titulaire: Biogemma, 75001 Paris (FR)
(72) Inventeur: PEREZ, Pascual, F-63450 Chanonat (FR); FLAMENT, Pascal, F-28700 Cinq Ormes Houville-la-Branche (FR)
(74) Mandataire: Bernasconi, Jean Raymond
(86) Numéro de dépôt international: PCT/FR1998/000381
(87) Numéro de publication internationale: WO 1998/038323

(56) Documents cités:
- WO-A-91/09957
- WO-A-92/11379
- WO-A-93/01283
- WO-A-93/02197
- WO-A-96/04393
- WO-A-96/15252
- ELLSTRAND N. AND HOFFMAN C.: "Hybridization as an avenue of escape for engineered genes" BIOSCIENCE, vol. 40, no. 6, juin 1990, pages 438-442, XP002048569 cité dans la demande

## Description

L'invention concerne de nouvelles utilisations de la stérilité mâle pour limiter les risques liés à la production de plantes transgéniques pour l'homme et l'environnement.

Les bénéfices de l'utilisation de plantes transgéniques sont très prometteurs. La transgenèse de gènes de mammifères dans une cellule végétale offre en particulier une voie de production en grandes quantités de protéines recombinantes nouvelles, à un coût de production réduit et sans risque de contaminations virales ou subvirales (prions).

Cependant, ces bénéfices ne doivent pas faire oublier les aspects de nature écologique liés à la production de plantes transgéniques, auxquels le public est très sensible.

La Demanderesse a présentement mis au point une technologie mettant la stérilité mâle au service d'une "biotechnologie verte", respectueuse des aspects écologiques et humains.

Une amélioration possible réside dans le contrôle de la dissémination du transgène dans l'environnement.

L'isolation spatiale des parcelles cultivées permet déjà de réduire le risque de "fuites" du transgène mais cette méthode est contraignante et devient problématique avec la multiplication des produits.

L'intérêt de méthodes génétiques pour isoler le transgène et réduire le risque de "fuite" a été suggéré dans un article d'Ellstrand en 1990. Parmi de nombreuses propositions de solutions face au problème de "fuite" du transgène, Ellstrand note que des génotypes mâle stérile pourraient êre introduits, ou qu'un gène létal pour le pollen pourrait être lié directement à un gène construit par génie génétique. Cependant, aucun développement technique n'avait été mis en oeuvre dans ce sens. Or, il était impossible de prévoir le succès de la mise en oeuvre d'un tel développement, en raison notamment du nombre de paramètres mis en jeu dans les techniques de génie génétique chez les plantes.

La Demanderesse a mis au point une technologie permettant d'empêcher la dissémination du transgène par voie pollinique et donc sa "fuite" dans l'environnement, technologie selon laquelle une plante mâle stérile (artificielle choisie parmi le maïs, le colza ou la tomate, comprenant un gène de stérilité mâle artificielle (gène AMS), dans le génome de laquelle est intégré un transgène d'intérêt dont l'expression n'est pas empêchée par la présence du gène AM est utilisée pour éviter la dissémination dudit transgène.

L'intérêt du contrôle de la fertilité mâle est connu pour la production de plantes hybrides qui implique le croisement de deux lignées différentes.

Une des méthodes pour empêcher l'auto-fécondation en contrôlant la pollinisation est la "castration" manuelle des organes mâles de la plante.

Des recherches sont également menées pour un contrôle génétique du développement du pollen.

La stérilité mâle peut être "acquise", c'est-à-dire qu'elle est indépendante d'une quelconque manipulation génétique par la voie de l'ADN recombinant. On peut distinguer la stérilité mâle cytoplasmique de la stérilité mâle nucléaire (Williams, 1995). La stérilité mâle cytoplasmique est liée à des changements dans l'organisation et l'expression du génome mitochondrial, la stérilité mâle nucléaire résulte de mutations dans le génome du noyau de la cellule.

La stérilité mâle peut aussi être "artificielle", c'est-à-dire qu'elle est induite par l'expression d'un gène conférant la stérilité mâle (gène AMS) qui est inséré soit dans le génome mitochondrial (stérilité mâle cytoplasmique), soit dans le génome nucléaire (stérilité mâle nucléaire).

La demande internationale WO 96/04 393 décrit notamment l'utilisation d'un gène de stérilité mâle AMS en tant que séquence bloquant l'expression d'un gène d'intérêt auquel le gène AMS est génétiquement lié, tout en permettant d'éviter l'autopollinisation des plantes transformées par ces séquences.

Conformément à l'invention, ladite plante mâle stérile artificielle choisie parmi le maïs, le colza ou la tomate utilisée pour éviter la dissémination d'un transgène d'intérêt intégré dans le génome de ladite plante est porteuse :
- soit d'une stérilité mâle cytoplasmique, de préférence une stérilité mâle artificielle induite par un transgène intégré dans le génome mitochondrial de ladite plante ;
- soit d'une stérilité mâle nucléaire, de préférence une stérilité mâle induite par un transgène inséré dans un site non essentiel du génome nucléaire de ladite plante.

De manière préférentielle, conformément à l'invention, ladite plante peut avoir été rendue mâle stérile par l'insertion, dans un site non essentiel du génome nucléaire, d'une séquence comprenant un gène AMS et ledit transgène d'intérêt génétiquement liés.

La liaison entre le gène d'intérêt et le gène de l'AMS aura pour effet d'interdire sa dissémination par voie pollinique, permettant ainsi une multitude de productions différentes dans un même environnement.

En outre, le transfert du gène de l'AMS lui-même vers les plantes de type sauvage n'est pas à craindre car il ne sera pas susceptible d'être retenu au sein d'une population sauvage dans la mesure où il représente plus un "fardeau génétique" qu'un quelconque avantage sélectif.

Par liaison entre gène d'intérêt et gène de l'AMS, on entend une distance génétique suffisamment faible pour que les fréquences de recombinaison au cours de la méiose soient négligeables.

Par transformation génétique, on peut introduire dans une plante deux voire trois gènes dont la liaison physique est absolue.

L'invention a également pour objet une plante transgénique, ou partie de plante transgénique choisie parmi le maïs, le colza ou la tomate, caractérisée en ce que ; transgène d'intérêt lié génétiquement à un gène de stérilise mâle artificielle ladite plante transgénique ou ladite partie de de plante transgénique comprend un (gène AMS) associé à des éléments permettant son expression dans les cellules végétales, notamment un promoteur et un terminateur de transcription, l'expression dudit transgène n'étant pas empêchée par la présence du gène AM.

Conformément à la présente invention, il est t entendu que la présence dudit gène AMS n'empêche pas l'expression dudit transgène d'intérêt.

Par "partie" de plante transgénique, on entend notamment feuilles, fruits, ou cellules de plantes transformées génétiquement.

Selon un mode de réalisation préféré de l'invention, la stérilité mâle artificielle peut être conférée par un gène constitué d'une séquence codant une protéine susceptible de dégrader les ARN cellulaires (RNAse) sous contrôle d'un promoteur anthère spécifique tels que décrit par PAUL W. et al. (1992).

Cette RNase peut être la Barnase de *Bacillus amyloliquefaciens.* Le promoteur peut avantageusement être le promoteur A9 *d'Arabidopsis thaliana* spécifique du tapis de l'anthère.

Les plantes exprimant ce gène chimérique deviennent incapables de produire un pollen viable grâce à la destruction spécifique des cellules du tapis de l'anthère. De telles plantes sont par ailleurs normales.

Les plantes selon l'invention étant incapables de produire du pollen viable, il n'y a aucun risque de dissémination des gènes de stérilité mâle et d'intérêt par le pollen.

La multiplication de ces plantes se fait en apportant du pollen provenant de plantes non porteuses du gène d'intérêt selon l'invention.

Dans le cas du maïs, la culture des plantes transgéniques en vue de la production peut être assurée selon un schéma de type production de semences (4 ou 6 lignes femelles suivies de 2 lignes pollinisatrices). Les plantes utilisées comme femelles (plantes porteuses du gène d'intérêt) sont semées en double densité. Dans un tel schéma, le gène d'intérêt est lié à un gène de sélection (par exemple, gène conférant une résistance à un herbicide), ce qui permet ensuite, par un traitement avec l'agent de sélection (herbicide par exemple), d'éliminer les plantes autres que mâles stériles. La récolte ne se fait alors que sur les lignes femelles.

Pour la production finale, un semis en mélange peut également être envisagé. Dans ce cas, la lignée pollinisatrice est non porteuse du gène d'intérêt et est utilisée en mélange (10 %) avec la lignée utilisée comme femelle. Le semis est réalisé en double densité. Le champ est dans ce cas récolté dans son intégralité.

Selon un autre mode de réalisation de l'invention, on utilise la stérilité mâle cytoplasmique pour limiter la dissémination d'un transgène intégré dans le génome d'une plante.

Les plantes porteuses d'une stérilité mâle cytoplasmique sont affectées dans leur génome mitochondrial, ce qui les rend inaptes à la production de pollen en absence des gènes nucléaires de restauration. Cette stérilité mâle peut être soit "acquise", soit provoquée artificiellement par l'insertion dans le génome mitochondrial d'un gène conférant la stérilité mâle cytoplasmique (gène CMS), par exemple le gène Ogura.

De telles plantes sont habituellement utilisées par les établissements semenciers pour faciliter leur production de semences. Une part importante de la production de semences de maïs repose, par exemple, en Europe, sur l'utilisation d'une stérilité mâle cytoplasmique dite de type C.

Selon un mode de réalisation préféré de l'invention, on introduit un transgène par rétrocroisement dans une lignée de plante, notamment de maïs, porteuse d'une stérilité mâle cytoplasmique. La plante mâle stérile est dans ce cas utilisée comme parent femelle. Deux rétrocroisements successifs suivis d'évaluations et sélections dans la descendance permettent d'atteindre l'état homozygote pour ce transgène.

En absence de gène nucléaire de restauration (gène Rf4 pour le cytoplasme C), la plante porteuse du transgène sera donc rendue mâle stérile. Aucun grain de pollen porteur d'un transgène ne sera donc émis dans l'environnement. Si la plante est homozygote pour le transgène, tous les grains récoltés sont porteurs du gène et donc expriment le phénotype.

La culture de ces plantes en vue de la production est réalisée en cultivant des plantes pollinisatrices à proximité des plantes transgéniques mâles stériles. Ceci peut être obtenu soit par mélange de semences (10 % de pollinisateur sont suffisants), soit par une alternance de lignes femelles (mâles stériles) et de lignes pollinisatrices. Dans ce dernier cas, seules les femelles sont récoltées.

L'invention a également pour objet un procédé de production d'un produit d'expression d'un transgène d'intérêt caractérisé en ce qu'il comprend :
a)
   - soit la transformation de cellules végétales de maïs, de colza ou de tomate, à l'aide d'un hôte cellulaire tel que défini précédemment, lui-même transformé par un vecteur contenant un transgène d'intérêt associé à des éléments permettant son expression dans les cellules végétales, lié génétiquement à un gène AMS associé à des éléments permettant son expression dans les cellules végétales, la présence dudit gène AMS n'empêchant pas l'expression dudit transgène d'intérêt de manière à intégrer dans le génome de ces cellules un gène AMS lié génétiquement à un transgène d'intérêt ;
   - soit la transformation de cellules végétales de maïs, de colza ou de tomate, porteuses d'une stérilité mâle cytoplasmique ou nucléaire de manière à intégrer un transgène d'intérêt dans le génome de ces cellules ;
b) la régénération de plantes transformées présentant un phénotype mâle stérile en exprimant ledit transgène d'intérêt à partir des cellules végétales transformées sus-mentionnées ;
c) la récupération du produit d'expression dudit transgène d'intérêt dans lesdites cellules ou plantes transformées sus-mentionnées, notamment par extraction, suivie, le cas échéant, par une purification.

Un autre aspect lié à la production de plantes transgéniques réside dans la présence de gènes éventuellement jugés indésirables, voire néfastes, pour l'homme et/ou l'environnement, en particulier aux yeux du public.

En effet, pour obtenir des plantes transgéniques, il faut trier parmi des millions de cellules, celles comportant la modification à introduire. Pour ce faire, on utilise des gènes dits marqueurs qui confèrent habituellement des résistances à des antibiotiques ou à des herbicides. Ces gènes sont parfois jugés indésirables et différentes stratégies (cotransformation, utilisation de recombinases...) ont été envisagées pour tenter de les éliminer.

Ainsi, la demande de brevet WO 92 01370 divulgue un procédé pour produire une plante transgénique contenant un gène d'intérêt, libérée de gènes marqueurs utilisant un système de transposition.

Par ailleurs, la demande WO 91 09957 divulgue un procédé de recombinaison site-spécifique dans des cellules de plantes qui utilise un système Cre/lox pour produire une délétion, une insertion ou un échange réciproque de segments d'ADN. Le procédé décrit est applicable à l'élimination d'un gène marqueur. Le déposant cite aussi l'utilisation de ce procédé pour restaurer la fertilité dans le cadre de la production de semences hybrides par génie génétique.

Cependant, dans l'art antérieur, la stérilité mâle n'avait pas été utilisée comme marqueur en tant que tel dans des opérations de criblage.

Selon un mode de réalisation, le gène AMS peut servir de marqueur positif pour le criblage de plantes ayant intégré un transgène d'intérêt, les individus possédant ledit gène AMS étant sélectionnés.

La présence de ce gène AMS peut être détectée notamment par des analyses moléculaires utilisant des réactions de polymérisation en chaîne (PCR), et/ou par des analyses de Southern blot selon des techniques usuelles (Sambrook et al., 1989). Les plantes possédant une stérilité mâle peuvent aussi être tout simplement sélectionnées en observant la présence ou l'absence de développement des grains de pollen. Le gène AMS ayant servi de marqueur positif peut ensuite être considéré comme indésirable et peut être excisé.

Selon un autre mode de réalisation de l'invention, le gène AMS permet l'amélioration du procédé d'élimination d'un fragment d'ADN exogène indésirable dans le cadre d'opérations de criblage de plantes transformées génétiquement.

En effet, pour être efficaces, les stratégies de criblage existantes, reposant sur l'utilisation d'un gène marqueur, telles que décrites dans les demandes de brevet précitées par exemple, doivent fonctionner à une fréquence très élevée. Les cellules ou plantes ayant perdu le gène marqueur ne sont plus sélectionnables par rapport à celles dont elles sont issues. Le tri repose alors sur des méthodes moléculaires lourdes et onéreuses.

La Demanderesse a présentement découvert l'intérêt de l'utilisation d'un gène conférant la stérilité mâle artificielle (gène AMS) comme "marqueur suicide" ou "négatif" d'un événement d'excision, à savoir que seuls les individus qui l'auront perdu se multiplient. Ceci permet l'utilisation de stratégies d'élimination d'un fragment d'ADN exogène indésirable ayant des rendements faibles et donc l'élargissement des champs d'investigation dans ce domaine.

Le gène AMS est lié génétiquement à un fragment d'ADN indésirable de telle sorte que ledit gène AMS et ledit fragment d'ADN indésirable puissent être excisés simultanément.

Ledit fragment d'ADN exogène indésirable peut être notamment un gène marqueur, de préférence un gène conférant une résistance à un antibiotique.

Les systèmes d'élimination de gènes indésirables reposent généralement sur deux composants: un fragment d'ADN excisable qui contient le gène indésirable et un inducteur de cette excision.

Selon un mode de réalisation on introduit dans le fragment excisable un gène conférant la stérilité mâle artificielle. Les plantes transformées avec ce premier composant seront donc mâles stériles, et maintenues par rétrocroisement ("back-cross"). L'inducteur apporté par un croisement, entraînera l'élimination du fragment d'ADN contenant le gène AMS, provoquant ainsi le développement de fruits issus d'autofécondation. Ces fruits contiennent des individus débarrassés du gène AMS et de gène indésirable.

En pratique, il suffit de recueillir les seules graines produites en autofécondation par des plantes F1 contenant les deux composants.

Selon un autre mode de réalisation la plante ayant intégré le fragment d'ADN excisable contenant le gène AMS peut être transformée par un fragment d'ADN inducteur de l'excision.

Le système d'excision utilisé conformément à l'invention pour éliminer le fragment d'ADN indésirable peut être un système de transposition, tel que notamment le système Ac/Ds du maïs, ou un système de recombinaison, tel que notamment le système Cre/lox du bactériophage P1, le système FLP/FRT de la levure, la recombinase Gin du phage Mu, la recombinase Pin de *E. coli* ou le système R/RS du plasmide pSR1.

Un vecteur, notamment plasmide, caractérisé en ce qu'il comporte un fragment d'ADN excisable qui comprend un fragment d'ADN indésirable et ledit gène AMS, ledit fragment d'ADN indésirable étant de préférence un gène marqueur, de préférence un gène conférant une résistance à un antibiotique, ledit gène AMS et ledit fragment d'ADN indésirable étant associés chacun respectivement à des éléments permettant leur expression dans les cellules végétales, notamment un promoteur et un terminateur de transcription, est utilisé pour la. transformation de cellules végétales.

La transformation de cellules végétales peut être réalisée par transfert des vecteurs susmentionnés dans les protoplastes, notamment après incubation de ces derniers dans une solution de polyéthylèneglycol (PG) en présence de cations divalents (Ca²⁺) selon la méthode décrite dans l'article de Krens et al., 1982.

La transformation des cellules végétales peut également être réalisée par électroporation notamment selon la méthode décrite dans l'article de Fromm et al., 1986.

La transformation des cellules végétales peut également être réalisée par utilisation d'un canon à gène permettant la projection, à très grande vitesse, de particules métalliques recouvertes des séquences d'ADN d'intérêt, délivrant ainsi des gènes à l'intérieur du noyau cellulaire, notamment selon la technique décrite dans l'article de Sanford, (1988).

Une autre méthode de transformation des cellules végétales, est celle de la micro-injection cytoplasmique ou nucléaire.

Selon un mode de réalisation particulièrement préféré du procédé de l'invention, les cellules végétales sont transformées par le vecteur susmentionné ledit hôte cellulaire étant susceptible d'infecter lesdites cellules végétales en permettant l'intégration dans le génome de ces dernières, des séquences d'ADN d'intérêt initialement contenues dans le génome du vecteur susmentionné.

Avantageusement, l'hôte cellulaire susmentionné utilisé est *Agrobacterium tumefaciens,* notamment selon les méthodes décrites dans les articles de Bevan, 1984 et d'An et al., 1986, ou encore *Agrobacterium rhizogenes,* notamment selon la méthode décrite dans l'article de Jouanin et al., 1987.

De manière préférentielle, la transformation des cellules végétales est réalisée par le transfert de la région T du plasmide circulaire extra-chromosomique inducteur de tumeurs Ti *d'Agrobacterium tumefaciens,* en utilisant un système binaire (Watson et al.).

Pour ce faire, deux vecteurs sont construits. Dans un de ces vecteurs, la région d'ADN-T a été éliminée par délétion, à l'exception des bords droit et gauche, un gène marqueur étant inséré entre eux pour permettre la sélection dans les cellules de plantes. L'autre partenaire du système binaire est un plasmide Ti auxiliaire, plasmide modifié qui n'a plus d'ADN-T mais contient toujours les gènes de virulence *vir,* nécessaires à la transformation de la cellule végétale. Ce plasmide est maintenu dans *Agrobacterium.*

Le gène AMS et le gène d'intérêt peuvent être associés ensemble ou chacun respectivement à un système de contrôle transcriptionnel, notamment un promoteur et un terminateur de transcription.

Le gène AMS peut être notamment associé à un système de contrôle transcriptionnel comprenant un promoteur permettant une expression spécifique dans l'anthère tel que le promoteur A3 ou A9 (WO 92 11379) ou les promoteurs TA29, TA26 ou TA13 (WO 89 10396).

Parmi les terminateurs de transcription pouvant être utilisés, on peut citer le terminateur polyA 35S du virus de la mosaïque du chou-fleur (CaMV), décrit dans l'article de Franck et al., (1980), ou le terminateur polyA NOS, qui correspond à la région en 3' non codante du gène de la nopaline synthase du plasmide Ti *d'Agrobacterium tumefaciens* souche à nopaline (Depicker et al., 1982).

Parmi les promoteurs de transcription pouvant être utilisés, par exemple en association avec le gène d'intérêt, on peut citer notamment :
- le promoteur 35S, ou avantageusement le promoteur constitutif double 35S (pd35S) du CaMV, décrits dans l'article de Kay et al., 1987 ;
- le promoteur PCRU du gène de la cruciférine de radis permettant l'expression des polypeptides recombinants de l'invention uniquement dans les semences (ou graines) de la plante obtenue à partir de cellules transformées selon l'invention, et décrit dans l'article de Depigny-This et al., 1992 ;
- les promoteurs PGEA1 et PGEA6 correspondant à la région 5' non codante des gènes de la protéine de réserve de graines, GEA1 et GEA6, respectivement, *d'Arabidopsis thaliana* (Gaubier et al., 1993) et permettant une expression spécifique dans les graines ;
- le promoteur chimérique super-promoteur PSP (Ni M et al., 1995), constitué de la fusion d'une triple répétition d'un élément activateur transcriptionnel du promoteur du gène de l'octopine synthase *d'Agrobacterium tumefaciens,* d'un élément activateur transcriptionnel du promoteur du gène de mannopine synthase et du promoteur mannopine synthase *d'Agrobacterium tumefaciens ;*
- le promoteur actine du riz suivi de l'intron actine de riz (PAR-IAR) contenu dans le plasmide pAct1-F4 décrit par Mc Elroy et al., 1991 ;
- le promoteur HMGW (High Molecular Weight Glutenine) d'orge (Anderson O.D. et al., 1989) ;
- le promoteur du gène de γzéine de maïs (Pγzéine) contenu dans le plasmide pγ63 décrit dans Reina et al., 1990, et permettant l'expression dans l'albumen des semences de maïs.

Avantageusement, le gène de la stérilité mâle et le gène d'intérêt sont associés à une ou plusieurs séquences codant pour un peptide responsable de l'adressage des polypeptides recombinants dans un compartiment déterminé de la cellule végétale, notamment dans le réticulum endoplasmique ou dans les vacuoles, ou bien même à l'extérieur de la cellule, dans la paroi pectocellulosique ou dans l'espace extracellulaire aussi appelé apoplasme.

Ces séquences codant pour un peptide d'adressage peuvent être d'origine végétale, humaine ou animale.

Parmi les séquences codant pour un peptide d'adressage d'origine végétale, on peut citer :
- la séquence nucléotidique de 69 nucléotides (indiqués dans les exemples ci-dessous) codant pour le prépeptide (peptide signal) de 23 acides aminés de la sporamine A chez la patate douce, ce peptide signal permettant l'entrée des polypeptides recombinants de l'invention dans le système de sécrétion des cellules végétales transformées selon l'invention (à savoir principalement dans le réticulum endoplasmique) ;
- la séquence nucléotidique de 42 nucléotides (indiquée dans les exemples ci-dessous) codant pour le propeptide N-terminal d'adressage vacuolaire de 14 acides aminés de la sporamine A chez la patate douce, permettant l'accumulation des polypeptides recombinants de l'invention dans les vacuoles des cellules végétales transformées selon l'invention ;
- la séquence nucléotidique de 111 nucléotides (indiquée dans les exemples ci-dessous) codant pour le prépropeptide de 37 acides aminés de la sporamine A constitué de la partie N-terminale vers la partie C-terminale des 23 acides aminés du peptide signal susmentionné suivis par les 14 acides aminés du propeptide susmentionné, ce prépropeptide permettant l'entrée des polypeptides recombinants de l'invention dans le système de sécrétion et leur accumulation dans les vacuoles des cellules végétales transformées selon l'invention ;
les trois séquences susmentionnées étant décrites dans les articles de Murakami et al., 1986 et Matsuoka et al., 1991 ;
- le propeptide carboxyterminal de la lectine d'orge décrit notamment dans les articles de Schroeder et al., 1993 et de Bednarek et al., 1991 ;
- et le PRS (Pathogenesis Related Protein, Cornelissen et al., 1986) permettant la sécrétion.

On peut également citer parmi les séquences codant pour un peptide d'adressage, celle codant pour les peptides KDEL, SEKDEL et HDEL à l'extrémité C-terminale et permettant un adressage dans le réticulum endoplasmique.

Les gènes d'intérêt à intégrer dans le génome de la cellule végétale peuvent être notamment des gènes codant pour des protéines d'origine humaine ou animale pouvant présenter un intérêt thérapeutique ou prophylactique, telles que le collagène, la lipase gastrique, etc.

Les gènes marqueurs utilisés peuvent être notamment des gènes conférant une résistance à des antibiotiques tels que l'hygromycine, la kanamycine, la bléomycine ou la streptomycine ou à des herbicides tels que le glufosinate, le glyphosate ou le bromoxynil.

### EXEMPLES

La construction des différents plasmides ainsi que leur ligation et la transformation des bactéries *Escherichia coli* DH5α rendues préalablement compétentes, est réalisée grâce aux techniques usuelles d'ADN recombinant (Sambrook et al., 1989).

### EXEMPLE 1

### Construction d'un plasmide binaire associant la stérilité mâle conférée par le gène codant la PR-glucanase, la production de la lipase gastrique de chien, la sélection sur kanamycine et la sélection sur basta, utilisable en trangenèse du colza.

Le plasmide "binaire" dérivé de pGA492 (An, 1986) qui contient entre les bordures droite et gauche; issues du plasmide pTiT37 *d'Agrobacterium tumefaciens,* sur son ADN de transfert, les séquences suivantes : le promoteur constitutif du gène nos codant pour la nopaline synthase (Depicker et al., 1982), la séquence codante du gène NPTII codant pour la néomycine phosphotransférase conférant la résistance à la kanamycine (Berg et Berg, 1983) délétée de la région des 8 premiers codons dont le codon initiateur méthionine ATG et fusionnée à la séquence des 14 premiers codons de la séquence codante du gène nos (Depicker et al., 1982), la séquence codante du gène nos dépourvue de la région des 14 premiers codons, le terminateur nos (Depicker et al., 1982), une région contenant des sites multiples de clonage (encore désignée polylinker) (HindIII-Xbacl-Sacl-Hpal-Kpnl-Clal-BglII) précédant le gène CAT codant pour la chloramphénicol acétyltransférase (Close et Rodriguez, 1982) et les séquences terminatrices du gène 6 du plasmide pTiA6 *d'Agrobacterium tumefaciens* (Liu et al., 1993).

### a) Construction du plasmide pBIOC500 portant le gène conférant la stérilité mâle.

Le gène chimérique correspondant à "promoteur A9-PR-glucanase-T35S" contenu dans pDW80PR (Worall et al., 1992) a été utilisé. Le fragment Kpnl-EcoRV portant ce gène chimérique a été isolé par double digestion enzymatique par Kpnl et EcoRV, purifié par électroélution après migration électrophorétique sur gel d'agarose à 0,8%, précipité à l'alcool, séché. Il a été inséré aux sites Kpnl et Smal du plasmide pBluescript KS+ commercialisé par Stratagene. La ligation a été réalisée avec 100 ng du plasmide déphosphorylé et de 50 ng de fragments KpnI-EcoRV dans un milieu réactionnel de 10 µl en présence de 1 µl de tampon T4 DNA ligase x 10 (Amersham) et de 2,5 U d'enzyme T4 DNA ligase (Amersham) à 14°C pendant 16 heures. Les bactéries *Escherichia coli* DH5α rendues préalablement compétentes, ont été transformées. L'ADN plasmidique des clones obtenus, sélectionnés sur milieu contenant 50 µg/ml d'ampicilline, a été extrait selon la méthode de la lyse alcaline et analysé par digestion enzymatique par des enzymes de restriction. A partir du plasmide obtenu, le fragment Kpnl - Sstl portant le gène chimérique décrit ci-dessus a été introduit aux sites Kpnl et Sstl de pGA492. L'isolement du fragment chimérique est réalisée avec les procédés usuels. La ligation a été réalisée avec 100 ng du vecteur déphosphorylé et de 50 ng de fragments portant le fragment Kpnl-Sstl dans un milieu réactionnel de 10 ml en présence de 1 µl de tampon T4 DNA ligase x 10 (Amersham) et de 2,5 U d'enzyme T4 DNA ligase (Amersham) à 14°C pendant 16 heures. Les bactéries *Escherichia coli* DH5α rendues préalablement compétentes, ont été transformées. L'ADN plasmidique des clones obtenus, sélectionnés sur milieu contenant 12 pglml de tétracycline, a été extrait selon la méthode de la lyse alcaline et analysé par digestion enzymatique par des enzymes de restriction. Le vecteur obtenu est appelé pBIOC500.

### b) Construction du plasmide pBIOC501 portant le gène conférant la résistance au Basta.

Le fragment EcoRI - Hindlll portant le gène chimérique "P35S - pat- TNOS", isolé à partir du plasmide plB16.1 (Broer et al., 1988), a été inséré aux sites EcoRI et HindIII de pBSIISK+ commercialisé par Strategene et modifié par l'ajout d'un site Kpnl dans le site Smal de la séquence polylinker de pBSIISK+. Le plasmide résultant est appelé pBIOC501. L'ADN plasmidique des clones obtenus, sélectionnés sur milieu contenant 50 µg/ml d'ampicilline, a été extrait selon la méthode de la lyse alcaline et analysé par digestion enzymatique par des enzymes de restriction.

### c) Construction du plasmide pBIOC502 portant le gène codant pour la lipase gastrique de chien.

Le gène chimérique correspondant à "PCRU-PSLGL-LGC - T35S" isolé à partir de pBIOC93 décrit dans la demande WO 9633277, est porté par le fragment obtenu par double digestion Sacl et Xhol, le site Sacl étant réparé par l'action de l'enzyme T4 DNA Polymérase (New England Biolabs) selon les recommandations du fabricant. Ce fragment a été inséré entre le site Apal traité par l'action de l'enzyme T4 DNA Polymérase et le site Xhol du plasmide pBIOC501. Le plasmide résultant est appelé pBIOC502. L'ADN plasmidique des clones obtenus, sélectionnés sur milieu contenant 50 µg/ml d'ampicilline, a été extrait selon la méthode de la lyse alcaline et analysé par digestion enzymatique par des enzymes de restriction.

### d) Construction du vecteur binaire pBIOC503.

A partir de pBIOC502, un fragment Kpnl portant les cassettes d'expression, "PCRU-PSLGL-LGC-T35S" et "P35S - pat - TNOS", a été isolé et ligué au site Kpnl de pBIOC500. Le plasmide résultant est appelé pBIOC503. L'ADN plasmidique des clones obtenus, sélectionnés sur milieu contenant 12 µg/ml d'ampicilline, a été extrait selon la méthode de la lyse alcaline et analysé par digestion enzymatique par des enzymes de restriction.

L'ADN plasmidique du plasmide pBlOC503 a été introduit par transformation directe dans la souche LBA4404 *d'Agrobacterium tumefaciens* selon le procédé de Holsters et al. (1978).

### EXEMPLE 2

### Construction d'un plasmide binaire associant la stérilité mâle conférée par le gène codant pour la barnase, la production de la lipase gastrique de chien, la sélection sur kanamycine et la sélection sur basta, utilisable en trangénèse du colza.

### a) Construction du plasmide pBlOC504 portant le gène conférant la stérilité mâle.

Le gène chimérique correspondant à "promoteur A9 barnase - T35S" contenu dans pWP173 (Paul et al., 1992) a été utilisé. Le fragment Kpnl - EcoRV portant ce gène chimérique a été inséré aux sites Kpnl et Smal du plasmide pBluescript KS+ commercialisé par Strategene. A partir du plasmide obtenu, le fragment Kpnl - Sstl portant le gène chimérique décrit ci-dessus a été introduit aux sites Kpnl et Sstl de pGA492. Le vecteur obtenu est appelé pBIOC504. L'ADN plasmidique des clones obtenus, sélectionnés sur milieu contenant 50 µg/ml d'ampicilline, a été extrait selon la méthode de la lyse alcaline et analysé par digestion enzymatique par des enzymes de restriction.

### b) Construction du plasmide pBIOC501 portant le gène conférant la résistance au Basta.

Le vecteur a été construit comme décrit dans l'exemple 1 b).

### c) Construction du plasmide pBIOC502 portant le gène codant pour la lipase gastrique de chien.

Le vecteur pBIOC502 a été construit comme décrit dans l'exemple 1 c).

### d) Construction du vecteur pBIOC505.

A partir de pBIOC502, un fragment Kpnl portant les cassettes d'expression, "PCRU-PSLGL-LGC-T35S" et "P35S - pat - TNOS", a été isolé et ligué au site Kpnl de pBIOC504. Le plasmide résultant est appelé pBIOC505. L'ADN plasmidique des clones obtenus, sélectionnés sur milieu contenant 50 µg/ml d'ampicilline, a été extrait selon la méthode de la lyse alcaline et analysé par digestion enzymatique par des enzymes de restriction.

L'ADN plasmidique du plasmide pBIOC505 a été introduit par transformation directe dans la souche LBA4404 *d'Agrobacterium tumefaciens* selon le procédé de Holsters et al. (1978).

### EXEMPLE 3

### Obtention de plants de colza transgéniques

Les graines de colza de printemps *(Brassica napus* cv WESTAR ou lignées Limagrain) sont désinfectées pendant 40 minutes dans une solution de Domestos à 15 %. Après quatre rinçages à l'eau stérile, les graines sont mises à germer, à raison de 20 graines par pot de 7 cm de diamètre et 10 cm de haut, sur du milieu minéral de Murashige et Skoog (Sigma M 5519) avec 30 g/l de saccharose et solidifié avec du gel Agar à 5 g/l. Ces pots sont placés dans une chambre de culture à 26°C avec une photopériode de 16H/8H et sous une intensité lumineuse de l'ordre de 80 µE. m⁻². S⁻¹.

Après cinq jours de germination, les cotylédons sont prélevés stérilement en coupant chaque pétiole environ 1 mm au-dessus du noeud cotylédonaire.

Parallèlement, une préculture *d'Agrobacterium tumefaciens* souche LBA4404, contenant les plasmides binaires, est réalisée en Erienmeyer de 50 ml, pendant 36 heures à 28°C dans 10 ml de milieu bactérien 2YT (Sambrook et al., 1989) complémenté avec les antibiotiques utiles à la sélection de la souche utilisée.

Cette préculture sert à ensemencer à 1 % une nouvelle culture bactérienne effectuée dans les mêmes conditions. Au bout de 14 heures, la culture est centrifugée 15 minutes à 3 000 g et les bactéries sont reprises dans un volume équivalent de milieu de germination liquide. Cette suspension est distribuée dans des boîtes de Pétri de 5 cm de diamètre à raison de 5 ml/boîte.

L'extrémité sectionnée du pétiole est immergée quelques secondes dans la solution d'agrobactéries ainsi préparées, puis le pétiole est enfoncé de quelques millimètres dans le milieu de régénération. Ce milieu a la même composition de base que le milieu de germination avec en plus 4 mg/l de benzyl-amino-purine (BAP), phytohormone favorisant la néoformation de bourgeons. Dix explants (cotylédon avec pétiole) sont mis en culture par boîte de Pétri de 9 cm de diamètre (Greiner référence 664102).

Après deux jours de coculture dans les mêmes conditions environnementales que les germinations, les explants sont repiqués dans des boîtes phytatray (Sigma, référence P1552) contenant le milieu précédent complémenté avec un agent sélectif : 45 mg/l de sulfate de kanamycine (Sigma, référence K 4000) et un bactériostatique : mélange de 1/6 en poids de sel de potassium d'acide clavulanique et de 5/6 sel de sodium d'amoxicilline (Augmentin® injectable) à raison de 600 mg/l.

Deux fois de suite, à trois semaines d'intervalle, les explants sont repiqués stérilement sur du milieu neuf dans les mêmes conditions.

Les bourgeons verts apparus à la fin du deuxième ou du troisième repiquage sont séparés de l'explant et mis en culture individuellement dans des pots transparents de 5 cm de diamètre et de 10 cm de haut contenant un milieu identique au précédent mais dépourvu de BAP. Après trois semaines de culture, la tige du bourgeon transformé est sectionnée et le bourgeon est repiqué dans un pot de milieu frais. Au bout de trois à quatre semaines, les racines sont assez développées pour permettre l'acclimatation de la plantule dans un phytotron. Les bourgeons qui ne sont pas verts ou enracinés sont éliminés. Ces plantules sont alors transplantées dans des godets de 7 cm de côté remplis de terreau (norme NF U44551 : 40 % de tourbe brune, 30 % de bruyère tamisée et 30 % de sable) saturé en eau. Après deux semaines d'acclimatation en phytotron (température : 21 °C ; photopériode :16H/8H et 84 % d'humidité relative), les plantules sont rempotées dans des pots de 12 cm de diamètre remplis du même terreau enrichi en engrais retard (Osmocote, à raison de 4 g/l de terreau) puis transportées en serre (classe S2) régulée à 18°C avec deux arrosages quotidiens de deux minutes à l'eau.

Dès l'apparition de fleurs, celles-ci sont ensachées (Crispac, référence SM 570y 300 mm*700 mm) de façon à empêcher la fécondation croisée.

Lorsque les siliques sont arrivées à maturité, elles sont récoltées, séchées puis battues. Les graines obtenues servent au dosage de l'activité biochimique du gène d'intérêt.

La sélection de la descendance transgénique se fait par germination sur un milieu contenant du sulfate de kanamycine à raison de 100 à 150 mg/l (selon les génotypes). Les conditions opératoires sont identiques à celles décrites ci-dessus, à ceci près que les germinations sont effectuées en tubes de verre avec une seule graine par tube. Seules les plantules développant des racines secondaires les trois premières semaines, sont acclimatées en phytotron avant d'être passés en serre.

### EXEMPLE 4

### Obtention de plantes de tabac transgéniques

Les plants de tabac utilisés pour les expériences de transformation *(Nicotiana tabacum* var. *Xanthi* NC et PBD6) sont cultivés *in vitro* sur le milieu de base de Murashige et Skoog (1962) additionné des vitamines de Gamborg et al (1968), Sigma référence M0404), de saccharose à 20 g/l et d'agar (Merck) à 8 g/l. Le pH du milieu est ajusté à 5,8 avec une solution de potasse avant autoclavage à 120°C pendant 20 minutes. Les plantules de tabac sont repiquées par bouture des entre-noeuds tous les 30 jours sur ce milieu de multiplication MS20.

Toutes les cultures *in vitro* sont réalisées en enceinte climatisée, dans les conditions définies ci-dessous :
- intensité lumineuse de 30 µE.m⁻².S⁻¹, photopériode de 16 heures ;
- thermopériode de 26°C le jour, 24°C la nuit.

La technique de transformation utilisée est dérivée de celle de Horsch et al. (1985).

Une préculture *d'Agrobacterium tumefaciens* souche LBA4404 contenant les plasmides binaires est réalisée durant 48 heures à 28°C sous agitation, dans du milieu LB (Sambrook et al., 1989) additionné des antibiotiques adéquats (kanamycine). La préculture est ensuite diluée au 1/50 dans le même milieu et cultivée dans les mêmes conditions. Après une nuit, la culture est centrifugée (10 minutes, 3 000 g), les bactéries sont reprises dans un volume équivalent de milieu MS30 (30 g/l saccharose) liquide et cette suspension est diluée au 1/10.

Des explants d'environ 1 cm² sont découpés à partir de feuilles de plantules décrites ci-dessus. Ils sont ensuite mis en contact de la suspension bactérienne pendant une heure, puis séchés rapidement sur papier filtre et placés sur un milieu de coculture (MS30 solide).

Après deux jours, les explants sont transférés en boites de Pétri sur le milieu de régénération MS30, contenant un agent sélectif, la kanamycine (2 000 mg/l), un bactériostatique, l'Augmentin® (400 mg/l) et les hormones nécessaires à l'induction de bourgeons (BAP, 1 mg/l et ANA, 0,1 mg/l). Un repiquage des explants est effectué sur le même milieu après deux semaines de culture. Après deux semaines supplémentaires, les bourgeons sont repiqués en boîtes de Pétri sur le milieu de développement composé du milieu MS20 additionné de kanamycine et d'Augmentin. Après quinze jours, les bourgeons sont repiqués de moitié. L'enracinement prend environ 20 jours, au terme duquel les plantules peuvent être clonées par bouture d'entre-noeuds ou sorties en serre.

### EXEMPLE 5

### Construction d'une source de transposase.

On a construit un plasmide binaire portant une source de transposase fixée sous le promoteur 35S, puisqu'il s'est avéré conduire une expression forte et précoce de cette transposase (Finnegan et al., 1993).

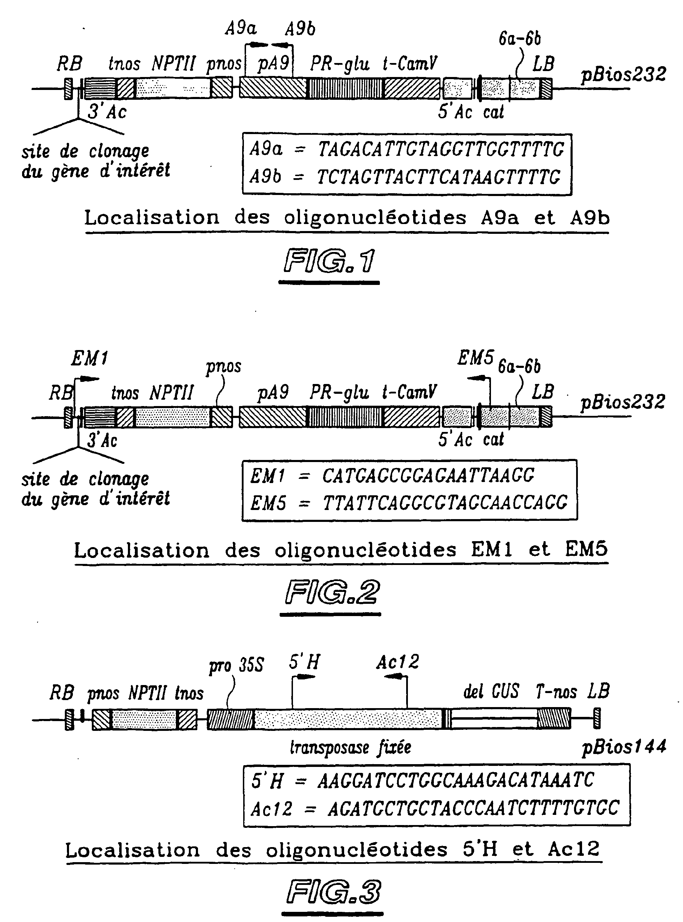

Pour ce faire, le fragment BamHI-EcoRI de pBI35S Ac (construit par Finnegan) a été cloné aux sites BamHI et SnaBI de pB1121 (Jefferson et al., 1987)). Le plasmide résultant nommé pBIOS144 contient, entre les bordures droite et gauche, le gène de résistance à la kanamycine NPTII sous promoteur et terminateur nos. L'élément Ac délété de sa partie 5' sous le promoteur 35S, constituant ainsi la source de transposase fixée, et 1400 paires de bases de la partie 5' du gène codant pour la β-glucuronidase d'*E. coli* (gène GUS noté del GUS sur la figure 3) suivi du terminateur nos.

### EXEMPLE 6

### Construction de l'élément Ds : Kana^{R}-AMS.

Les étapes successives ayant conduit à la fabrication de ce vecteur sont les suivantes :

### a) Construction du plasmide pBIOC203 portant le gène conférant la résistance à la kanamycine.

On a inséré le fragment BamHI de 1 Kb (correspondant au gène NPTII) de pCamVNEO (Fromm et al., 1986) au site BamHI de pBIOS1 (Perez et al., 1989). Le vecteur pBIOS 1 K résultant a été digéré par EcoRI. Le fragment EcoRI de BIOS 1 K a été rendu franc par la polymérase Klenow, puis cloné dans le plasmide AF 3'Ac (Cocherel et al., 1996) au site EcoRI constituant ainsi pBIOS203.

### b) Construction du plasmide pBIOS208 portant le gène AMS.

Le fragment EcoRl du vecteur pBGS phléo contenant 399 paires de bases de l'extrémité 5' de l'élément Ac (Cocherel et al., 1996) a été cloné dans pBSsk (Stratagene) au site EcoRl, constituant ainsi pBIOS204.

Le fragment EcoRI - Hindlll (comprenant l'extrémité 3' de Ac et la cassette NPTII) de pBlOS203 a été cloné dans pBSsk aux sites EcoRi - Hindlll constituant ainsi pBlOS205.

Le fragment Smal de pBIOS204 (comprenant l'extrémité 5' de Ac) a été cloné dans pBIOS205 au site Smal constituant ainsi pBIOS206.

Le fragment Sphl de DW 80 bin PR-glucanase contenant le gène PR-glucanase (PR-Glu) sous promoteur A9 et terminateur CaMV (Worall et al., 1992) a été cloné au site EcoRI de pBIOS206. Dans le plasmide résultant pBIOS208, le gène A9-PR-glucanase est inséré dans le sens inverse au gène NPTII.

### c) Insertion de l'élément Ds::Kana^{R}-AMS dans un vecteur binaire.

Le vecteur pGA 492DL a été obtenu après les modifications suivantes de pGA 492 (G. An) :
- délétion du fragment Sacll-Clal correspondant à la cassette d'expression du gène chimérique NPTII. Ce nouveau vecteur est appelé pGa 492D ;
- remplacement du fragment BgIII-Scal (perte d'une partie du gène CAT) de pGA 492D par le polylinker Sacl-Kpnl de pBSllsk. Ce nouveau vecteur s'appelle pGA 492 DL.

Le fragment HindlII-SpeI de pBIOS208 a été cloné aux sites HindlII-SpeI de pGA 492 DL pour constituer le plasmide binaire pBIOS232. La structure de cet ADN-T est schématisée dans les figures 1 et 2.

### EXEMPLE 7

### Transformation et sélection de la tomate

### a) Transformation de tomate (variété UC82B) par le vecteur binaire construit.

Le vecteur binaire pBIOS232 a été transféré dans la souche *d'Agrobacterium* LBA 4404, par conjugaison triparentale selon la technique décrite par Ditta et al..

La transformation de la tomate a été effectuée selon la technique modifiée de J. Fillatti :

Des graines du cultivar UC82B sont stérilisées 15 minutes dans 10 % de Domestos, et rincées trois fois dans de l'eau stérile.

Elles sont ensuite semées en pot contenant du milieu de culture MSSV/2 pendant sept ou huit jours.

Les cotylédons sont prélevés et découpés transversalement en trois parties. Seule la partie centrale est conservée et mise en culture à 26°C et à la lumière, sur milieu KCMS additionné d'Acétosyringone face inférieure contre le milieu.

Des bactéries de la souche *Agrobacterium* LBA 4404 sont mises en culture une nuit dans du milieu LB supplémenté avec l'agent sélectif approprié (tétracycline). Le lendemain, la culture est centrifugée et le culot obtenu est repris dans du milieu KCMS liquide.

Les explants sont trempés 30 minutes environ dans une solution *d'Agrobacterium* diluée au 1/20 dans du milieu KCMS additionné d'Acétosyringone. Ils sont ensuite séchés rapidement sur papier filtre.

Les explants sont ensuite :
- remis sur le même milieu KCMS pendant deux jours à l'obscurité à 26°C ;
- lavés dans du milieu liquide 2Z additionné d'Augmentin (400 mg/l) et séchés sur papier filtre ;
- transférés sur milieu solide 2Z contenant 400 mg/l d'Augmentin et 100 mg/l de Kanamycine ;
cultivés à la lumière à 26°C ; et
- repiqués sur du milieu 2Z frais, quinze jours après.

Trois semaines après la coculture, les premiers bourgeons apparaissent. Lorsqu'ils atteignent environ 1 cm, ils sont séparés de l'explant et repiqués sur milieu Dev où ils s'enracinent en une ou deux semaines s'ils sont bien transformés.

Quand ils sont bien enracinés, ils sont repiqués en motte Jiffy-7 (par AS Jiffy Products) au phytotron, où ils s'acclimatent rapidement.

Dès que le système racinaire est bien développé en "Jiffy", les plantes sont repiquées en pot de 12 cm de diamètre puis en container de 30 cm de diamètre, et cultivées en serre, sous arrosage automatique et solution nutritive (solution diluée à 5 kg/50 l délivré à 1,6 %).

### b) Analyse moléculaire des transformants primaires contenant l'ADN-T de pBIOS144 ou pBIOS232.

Quand les plantes transformées sont assez développées en serre, des feuilles sont prélevées (environ 5 g) pour en extraire l'ADN analysé ensuite par Southern Blot selon les méthodes couramment utilisées (Sambrook et al.).

L'ADN de chaque plante est digéré avec l'enzyme Hindlll. Après migration et transfert sur membrane de nitrocellulose, ceux-ci sont soumis à des hybridations avec différentes sondes permettant de vérifier :
- le nombre de copies de l'ADN-T insérées,
- si l'ADN-T a été inséré dans son intégralité,
- la présence ou l'absence d'extrabordures.

Au terme de ces analyses moléculaires, on sélectionne uniquement les plantes comportant un ADN-T entier unique sans extrabordure.

Le même protocole a été mise en oeuvre pour transformer des cellules de tomate avec le plasmide pBIOS144 et sélectionner les plantes transformées.

### EXEMPLE 8

### Evaluation de la source de transposase fixée

Une fois la source de transposase fixée et intégrée dans les plantes, on a vérifié qu'elle était capable d'activer un élément "Ds". Pour cela, les plantes ayant le plasmide pBIOS144 sélectionnées sont croisées avec une plante contenant un élément Ds testeur dont l'excision restaure l'activité du gène Gus (β-glucuronidase). Ce Ds a été construit en deux étapes:
- le fragment BglII-SpeI de pBIOS206 correspondant au Ds::Kana^{R} a été inséré aux sites BamHI-Xbal de pBI221 (Clontech) pour obtenir pBIOS226.

On a ensuite inséré le plasmide pBIOS226 entier au site HindlII dans pGA 492 DL. Le plasmide binaire résultant pBIOS228 comporte entre les bordures droite et gauche le Ds::Kana^{R} entre le promoteur 35S et la partie codante du gène Gus.

Toute excision de ce Ds::Kana^{R} se traduira par une expression du gène rapporteur Gus. Un test Gus (Jefferson et al.) est réalisé sur les descendants F1 de ce type de croisement pour évaluer l'efficacité des différentes lignées contenant la transposase. Des taches bleues ont été détectées sur des cotylédons et des secteurs sur des feuilles de plantes issues de croisement entre une lignée "transposase" et une lignée "Ds testeur". Le nombre de taches et secteurs sur une plante atteste de l'effficacité de la source de transposase dans cette plante.

### EXEMPLE 9

### Génération de lignées de transposase fixée

Les transformants unicopie et sans extrabordure sélectionnés et évalués positivement selon l'exemple précédent ont été soumis à deux cycles sucessifs d'autofécondation. Les lots de graines T2 ont été récoltés séparément.

Pour chaque T2, on a évalué la ségrégation pour la résistance à la kanamycine afin de détecter les lots de graines homozygotes. Pour chaque lot, une trentaine de graines sont semées en terre en serre. Dix-huit jours après le semis, une solution à 400 mg/l de kanamycine est pulvérisée sur les plantules pendant trois jours. (Weide et al.,). Cinq jours après, il est aisé d'identifier les plantes sensibles à la kanamycine. D'après la ségrégation des plantes résistantes et sensibles, et conformément à la ségrégation Mendelienne d'un gène dominant, on identifie les lots de plantes homozygotes pour le transgène. Ce sont ceux-la que l'on garde préférentiellement et qui serviront à croiser les plantes contenant l'élément Ds::Kana^{R} -AMS.

### EXEMPLE 10

### Croisement des lignées Ds::Kana^{R} -AMS avec des lignées "transposase" homozygotes

Les transformants primaires sélectionnés (unicopie sans extrabordure) sont évalués dès leur floraison en serre, pour la fertilité par observation microcospique de coupe transversale du tube d'anthère de une ou deux fleurs par plante, dans du carmin acétique. A l'issue de cette évaluation, seules les plantes mâles stériles qui ne présentent pas de pollen sont conservées.

Les plantes stériles portant le Ds::Kana^{R} - AMS sont fécondées avec du pollen de lignées exprimant la transposase d'Ac. Les fruits sont récoltés et les graines extraites.

### EXEMPLE 11

### A - Identification d'événement d'excision sur les plantes F1

Les graines issues de croisement entre les plantes portant la source de transposase et celles portant le Ds::Kana^{R} -AMS sont semées en terreau en serre. Au stade cotylédons, un cotylédon par plantule est prélevé en vue d'une extraction rapide d'ADN (Lassner et al., 1989) pour un test PCR avec les oligonucléotides A9a et A9b (fig. 1). Les conditions de PCR utilisées sont les suivantes :
ADN : 40 ng (ou 10 µl de la microextraction)
oligo A9a (10 pM/µl) : 3 µl
oligo A9b (10 pM/µl) 3 µl
Tampon x 10 : 10 µl
Mix dNTP (5 mM) : 4 µl .
Taq Promega 0,4 µl
MgCl₂ 25 mM : 8 µl
H₂O up qsp 100 µl

Les réactions sont effectuées dans la machine Perkin 9600.

Après 2 minutes de dénaturation à 95°C, on réalise 40 cycles des opérations suivantes :
30"de dénaturation à 94°C
30" d'hybridation à 55°C
1'30" d'élongation à 72°C

Le but de ce test est d'identifier rapidement les plantes porteuses du gène AMS (bande à 800 paires de bases en PCR) de celle qui ne le portent pas (pas de bande en PCR). Les plantes porteuses du gène AMS sont rempotées et pendant leur floraison, on surveille l'apparition de fruits qui révéleraient un ou des secteurs d'excision somatique. L'observation de fruits sur des plantes F1 révèle soit une excision sans réinsertion du Ds soit une excision suivie d'une réinsertion avec perte d'activité.

Pour vérifier cela, les graines issues de ces fruits sont extraites et semées. Une analyse moléculaire par PCR de ces plantes est réalisée pour vérifier qu'elles ne portent plus le Ds::Kana^{R} -AMS, mais qu'elles possèdent la "cicatrice" de l'ADN-T et/ou le gène d'intérêt. Pour cela, un cotylédon de chaque plantule est prélevé pour extraction rapide d'ADN.

Un aliquot de cet ADN est prélevé pour être soumis à deux réactions PCR :
- l'une avec les oligonucléotides EM1 et EM5 (fig. 2),

Les conditions de PCR utilisées sont les suivantes :
ADN : 40 ng (ou 10 µl de la microextraction)
oligo EM1 (10 pM/µl) : 3 µl
oligo EM5 (10 pM/µl) : 3 µl
Tampon x 10 : 10 µl
Mix dNTP (5 mM) : 4 µl
Taq Promega : 0,4 µl
MgCl₂ 25 mM : 18 µl
BSA : 8 µl
Glycérol : 2,5 µl
H₂O up qsp 100 µl

Les réactions sont effectuées dans la machine Perkin 9600.

Après 2 minutes de dénaturation à 95°C, on réalise 40 cycles des opérations suivantes :
30"de dénaturation à 94°C
30" d'hybridation à 62°C
45" d'élongation à 72°C

Ce test permet de voir si la plantule est porteuse de la cicatrice de l'ADN-T et/ou du gène d'intérêt. Dans le cas où l'élément Ds est excisé, on attend un fragment de 300 paires de bases environ, pour la cicatrice seule, auquel il faut rajouter la taille du gène d'intérêt ;
- l'autre avec les oligonucléotides 5'H et Ac12 (fig. 3).

Les conditions de PCR utilisées sont les suivantes :
ADN : 40 ng (ou 10 µl de la microextraction)
oligo 5'H (10 pM/µl) : 3 µl
oligo Ac12 (10 pM/µl): 3 µl
Tampon x 10 : 10 µl
Mix dNTP (5 mM) : 4 µl
Taq Promega : 0,4 µl
MgCl₂ 25 mM : 18 µl
BSA x 100 : 8 µl
Glycérol : 2,5 µl
H₂O up qsp 100 µl

Les réactions sont effectuées dans la machine Perkin 9600.

Après 2 minutes de dénaturation à 95°C, on réalise 40 cycles des opérations suivantes :
30"de dénaturation à 94°C
30" d'hybridation à 62°C
1'30" d'élongation à 72°C

Ce test permet de vérifier l'absence du gène codant pour la transposase. S'il n'y a pas de fragment amplifié, la transposase est absente. Si elle est présente, on détecte une bande de 1,6 Kb.

Les plantes issues d'événements d'excision ne portant plus que le gène d'intérêt sans la résistance à la kanamycine ni l'ADN-T portant le gène codant pour la transposase seront positives avec le premier jeu d'oligonucléotides (EM1-EM5) et négatives avec le deuxième (5'H-Ac12).

Une analyse Southern réalisée sur ces plantes permet de confirmer la présence du gène d'intérêt et l'absence du gène de résistance à la kanamycine.

### B - Identification de transformants dépourvus du marqueur sélectif

### 1) Matériels

### Sonde NPTII

Amplification d'un fragment de 1 KB du gène NPTII du vecteur pBios232 avec les oligonucléotides Kana7 et Kana8, de séquences.
Kana 7 : gctcgacgttgtcactgaag
Kana 8 : cccggaaaacgattccgaag

Le gène NPTII utilisé a été isolé à partir du transposon Tn5 *d'Escherichia coli* (Berg et Berg, 1983).

### Sondes Cat intraLB +intra RB :

### Mélange de 2 sondes :

Cat int LB : fragment Sall de l'ADN-T de pGA 492 DL contenant la bordure gauche et une partie du gène Cat
int RB ; fragment Sall de l'ADN-T de pGA 492 contenant la bordure droite

L'ensemble des 2 sondes correspond à l'ensemble de l'ADN-T de pGA 492 DL qui est le vecteur de base ayant servi à la construction de pBios 232 ayant servi à créer les plantes Ds : :Kana^{R-}AMS.

### 2) Identification et culture des plantes à deux composants actifs :

Des graines F1 issues de croisement entre une plante portant le Ds: Kana^{R}-AMS et une plante portant la source de transposase fixée ont été obtenue et semées. Un test PCR sur de l'ADN de cotylédons de 173 plantes a été fait avec les oligonucléotides EM1 et EM5 comme décrit plus haut. Ce test a permis d'identifier 18 plantes où une bande de 350 pb a été amplifiée par EM1-EM5, révélant ainsi des événements d'excision somatiques, attestant la présence et la fonctionalité des 2 éléments du système.

Le système a été mis au point avec un ADN-T portant un Ds : :Kana^{R}- AMS mais pas de gène d'intérêt. De ce fait la bande d'excision révélée par un amplification faite avec EM1 et EM5 est d'environ 350 pb. Dans le cas où un gêne d'intérêt est inséré, cette bande sera allongée de la taille de ce gène. Selon un autre mode de réalisation, on peut également utiliser des oligonucléotides spécifiques du gène d'intérêt.

Ces 18 plantes ont été cultivées en champ pendant 4 mois . L'observation des fleurs est faite régulièrement pour vérifier la fertilité des anthères, ainsi que l'apparition de fruits. Pour 3 de ces 18 plantes des fruits sont apparus sur certains bouquets seulement. Ces plantes étaient donc chimériques pour les phénotypes mâle stérile /mâle fertile. Les fruits sur ces plantes ont été récoltés bouquet par bouquet en notant la hiérarchie du bouquet par rapport à la plante entière.

Des graines de fruits apparus sur 3 bouquets des 3 plantes redevenues partiellement fertiles ont été semées. Des feuilles - des plantules obtenues (4 plantules / bouquet), ont été prélevées en vue d'une extraction d'ADN pour une analyse de type Southern. L'ADN est soumis à une digestion avec l'enzyme Hind III (site unique dans l'ADN-T), avant d'être soumis à une électrophorèse et un transfert sur membrane. Les 36 échantillons correspondant aux descendants F2 ont été traités en même temps que de l'ADN des parents de l'hybride (la plante portant le Ds : :Kana^{R}-AMS et la plante portant la source de transposase fixée), de manière à avoir les profils de référence. L'hybridation du Southern blot avec les sondes NPTII et Cat-int LB a permis d'identifier des plantes qui ne portaient plus l'ADN-T contenant la source de transposase ni le Ds : : Kana^{R}-AMS mais qui contenaient bien la cicatrice de l'ADN-T, grâce à la comparaison avec le profil des parents..

Nous avons analysé au total 36 plantes, sans sélection préalable à la kanamycine, provenant de 3 bouquets des 3 plantes où des fruits ont été observés.

### 3) Bilan de l'analyse :

20 plantes portant l'ADN-T « cicatrisé » c'est à dire révélant 1 bande hybridant avec la sonde Cat int LB-RB, de taille inférieure à celle présente dans le parent portant le Ds. Cette même bande n'est pas hybridée par la sonde NPTII.

31 plantes portant la source de transposase, c'est à dire révélant une bande hybridant avec la sonde NPTII de taille identique à celle révélée par la même sonde chez le parent portant la source de transposase fixée.

5 plantes portant l'ADN-T vide mais ne portant pas la source de transposase.

Sur 36 plantes analysées, 5 se sont avérées être des transformants propres ne portant plus que l'ADN-T porteur du gène d'intérêt, et ayant excisé le gène marqueur de sélection.

II est donc possible de sélectionner des plantes transformées propres 2 générations après le transformant primaire.

Les avantages de cette technique sont multiples. Elle permet d'identifier de manière précoce les transformants dépourvus de marqueur sélectif.

Le croisement du transformant primaire avec la source de transposase est facilité et plus fiable puisque celui ci est mâle stérile.

Cette technique permet enfin le criblage d'un faible nombre de plantules pour identifier des transformants propres : sans présélection préalable, après analyse de 36 descendants de 3 plantes, il a été identifié 5 plantes contenant l'ADN-T dépourvu du Ds : : Kana^{R}- AMS.

Par ailleurs, il est possible d'améliorer l'identification des transformants dépourvus de marqueurs :
si on traite préalablement à la Kanamycine les descendants des fruits produits sur une plante portant le Ds : :Kana^{R}- AMS et la source de transposase, on doit avoir un quart de plantes Kana^{S} au lieu de 15/16 (normalement obtenus s'il n'y avait pas excision du Ds). Parmi ceux-ci, ¾ sont porteurs de l'ADN-T « propre », l'autre quart correspondant aux ségrégants classiques qui ne portent aucun transgène. L'observation d'une ségrégation ¾ de plantes résistantes pour ¼ de sensibles permet en outre de confirmer rapidement que le système d'élimination a été efficace.

Il est donc possible d'identifier des descendants propres en analysant très peu de plantes parmi les plantules F2 sensibles à la Kanamycine.

### 41 Conclusion :

Cette étude montre que l'adjonction d'un gène conférant la stérilité mâle artificielle au système d'élimination de gène marqueur par Ac/Ds, permet d'identifier rapidement les descendants portant le gène d'intérêt sans le marqueur sélectif, tout en facilitant le processus et en prévenant la dissémination par le pollen d'événements transgéniques insuffisamment caractérisés.

### EXEMPLE 12

### Construction d'un plasmide binaire pBIOC4-FLP portant la recombinase FLP.

La séquence codant la recombinase FLP de *Saccharomyces cerevisiae* est contenue dans pOG44 commercialisé par Stratagene. Le plasmide pOG44 a été digéré par les enzymes Hindlll et Kpnl et le fragment HindlII-KpnI a été isolé. La ligation a été réalisée avec 100 ng du vecteur pBIOS512 déphosphorylé et 50 ng de fragment HindIII-KpnI portant la séquence codant la recombinase dans un milieu réactionnel de 10 µl en présence de 1 µl de tampon T4 DNA ligase x 10 (Amersham) et de 2,5 U d'enzyme T4 DNA ligase (Amersham) à 14°C pendant 16 heures. Les bactéries *Escherichia coli* DHSα, rendues préalablement compétentes, ont été transformées. Le plasmide résultant est appelé pBIOS512-FLP. L'ADN plasmidique des clones obtenus, sélectionnés sur milieu contenant 50 µg/ml d'ampicilline, a été extrait selon la méthode de la lyse alcaline et analysé par digestion enzymatique par des enzymes de restriction. Le fragment portant la séquence codant la recombinase FLP a été isolé par digestion enzymatique par Kpnl suivi de l'action de l'enzyme T4 DNA Polymérase (New England Biolabs) selon les recommandations du fabricant puis de la digestion par Hindlll. Le fragment a été purifié par électrophorèse sur gel d'agarose 0,8%, électroélué, précipité à l'alcool et séché. Il a été introduit au site BamHl, traité par l'enzyme Klenow (New England Biolabs) selon les recommandations du fabricant, et au site HindlII du plasmide pBIOS512. La séquence nucléotidique de FLP a été vérifiée par séquençage à l'aide du kit de séquençage T7^{™} commercialisé par Pharmacia selon la méthode des didésoxynucléotides (Sanger et al., 1977). Le plasmide pBIOS512 dérive de pUC et porte la cassette d'expression "promoteur double 35S (PD35S) - terminateur NOS (TNOS)" (fragment EcoRI). Le promoteur double 35S provient de pJIT163 (WO 96 33277).

A partir de pBIOS512-FLP, le fragment EcoRI portant la cassette d'expression "PD35S-FLP-TNOS" a été isolé par digestion enzymatique par EcoRI, purifié sur gel d'agarose à 0,8%, électroélué, précipité à l'alcool et séché. Il a été introduit au site EcoRI de pBIOC4 (WO 96 33277) déphosphorylé par l'enzyme phosphatase alcaline d'intestin de veau (Boehringer Mannheim) selons les recommandations du fabricant. La ligation a été réalisée avec 100 ng du vecteur déphosphorylé et 50 ng de fragments portant la cassette "PD35S-FLP-TNOS" dans un milieu réactionnel de 10 µl en présence de 1 µl de tampon T4 DNA ligase x 10 (Amersham) et de 2,5 U d'enzyme T4 DNA ligase (Amersham) à 14°C pendant 16 heures. Les bactéries *Escherichia coli* DH5α rendues préalablement compétentes, ont été transformées. Le plasmide résultant est appelé pBIOC4-FLP. L'ADN plasmidique des clones obtenus, sélectionnés sur milieu contenant 12 µg/ml de tétracycline, a été extrait selon la méthode de la lyse alcaline et analysé par digestion enzymatique par des enzymes de restriction. L'ADN plasmidique du plasmide binaire pBIOC4-FLP a été introduit par transformation directe dans la souche LBA44O4 *d'Agrobacterium tumefaciens* selon le procédé de Holsters et al. (1978). La validité du clone retenu a été vérifiée par digestion enzymatique de l'ADN plasmidique introduit.

### EXEMPLE 13

### Construction d'un plasmide binaire et pBIOC511, portant la stérilité mâle et la sélection sur kanamycine entre des sites de recombinaison spécifique FRT, utilisable en trangénèse du tabac.

Le plasmide binaire pGA492 (An, 1986) a été délété des séquences suivantes : le promoteur constitutif du gène nos codant pour la nopaline synthase (Depicker et al., 1982)., la séquence codante du gène nptlI codant pour la néomycine phosphotransférase II (Berg et Berg, 1983) délétée de la région des 8 premiers codons dont le codon intiateur méthionine ATG et fusionnée à la séquence des 14 premiers codons de la séquence codante du gène nos (Depicker et al., 1982), la séquence codante du gène nos dépourvue de la région des 14 premiers codons, le terminateur nos (Depicker et al., 1982). Cette délétion a été réalisée par digestion totale Clal, suivie d'une digestion partielle Sacll. Le fragment est purifié, puis soumis à l'action de l'enzyme T4 DNA Polymérase (New England Biolabs) selon les recommandations du fabricant. La ligation du plasmide et la transformation de bactéries *Escherichia coli* DH5α rendues préalablement compétentes, sont réalisées en suivant les procédés usuels (Sambrook et al., 1989). Le plasmide résultant est appelé pBIOC506.

Le site d'intégration spécifique FRT a été amplifié par PCR à partir du plasmide pOG45 commercialisé par Strategene. Les deux oligodésoxynucléotides utilisés comme amorces pour la PCR sont : 5' CCC CTG CAG TTT TCA AAA GCG CTC TGA AGT TC 3' et 5' CCA AAG CTT GAA TTC GCC AGG GGG ATC TTG AAG TTC 3'.

Les fragments correspondants au site FRT, issus de l'amplification PCR ont été digérés par Pstl, soumis à l'action de l'enzyme T4 DNA Polymérase (New England Biolabs) selon les recommandations du fabricant, puis digérés par EcoRI. Ils ont été purifiés sur gel d'agarose à 2 %, électroélués, précipités à l'alcool, séchés et repris dans de l'eau. Puis ils ont été ligués entre le site Sacl préalablement soumis à l'action de l'enzyme T4 DNA Polymérase et le site EcoRl du plasmide pUC18 commercialisé par Pharmacia. Le plasmide résultant est appelé pBIOC507.

Un deuxième site FRT a été introduit dans le plasmide pBIOC507. Ce deuxième site FRT résulte de l'amplification PCR à l'aide des deux oligodésoxynucléotides :
5' CCC CTG CAG TTT TCA AAA GCG CTC TGA AGT TC 3' et
5' AAA GGT ACC GCC AGG GGG ATC TTG AAG TTC 3'.

Les fragments amplifiés ont digérés par Pstl et Kpnl, soumis à l'action de l'enzyme T4 DNA Polymérase, et ligués au site Sphl préalablement traité par l'action de l'enzyme T4 DNA Polymérase, du plasmide pBlOC507. Le plasmide résultant est appelé pBlOC508 et contient les deux sites FRT orientés dans le même sens.

A partir du plasmide pBIOC508, le fragment Hindlll - EcoRl portant les deux sites FRT, a été isolé. Ce fragment a été inséré aux sites HindIII et EcoRl du plasmide pBIOC506. Le plasmide résultant est appelé pBIOC509.

Pour conférer la stérilité mâle, le fragment Sphl portant le gène chimérique correspondant à "promoteur A9 - barnase -T35S" contenu dans pWP173 (Paul et al., 1992) a été utilisé. Ce fragment Sphl a été traité par l'action de l'enzyme T4 DNA Polymérase et ligué au site Pstl traité par l'action de l'enzyme T4 DNA Polymérase du plasmide pBIOC509. Le plasmide résultant est appelé pBIOC510.

Enfin, pour permettre la sélection sur kanamycine (Fromm et al., 1986), le fragment EcoRl traité par l'action de l'enzyme T4 DNA Polymérase, correspondant à la cassette d'expression "PNOS - nptII-TNOS" isolée à partir d'un plasmide de pBIOS1 dans lequel a été cloné au site BamH1 le fragment BamH1 codant pour le gène nptll, a été ligué au site Kpnl traité par l'action de l'enzyme T4 DNA Polymérase du plasmide pBIOC510. Le plasmide résultant est appelé pBIOC511.

### MILIEUX DE CULTURE

### LB

10 g/l Bactotryptone
5 g/l extrait de levure
10 g/l NaCl
pH 7,5 ajusté avec NaOH

### MSSV/2

2,2 g macro et micro-éléments de Murashige et Skoog (ref. M 6899 Sigma)
2 ml/l vitamines de Nitsch
15 g/l saccharose
8 g/l Agar-Agar
pH 5, 9 avant autoclavage

### 2Z

4,4 g macro et micro-éléments de Murashige et Skoog (ref. M 6899 Sigma)
2 ml/l vitamines de Nitsch
30 g/l saccharose
2 mg/l Zéatine
400 mg/l Augmentin
100 mg/l Kanamycine
8 g/l Agar-Agar
pH 5,9 avant autoclavage

### KCMS

4,4 g macro et micro-éléments de Murashige et Skoog (ref. M 6899 Sigma)
0,9 mg/l Thiamine
200 mg/l potassium dihydrogénophosphate
30 g/l saccharose
8 g/l Agar-Agar
200 µM Acétosyringone
0,2 mg/l 2-4D
0,1 mg/l Kinétine
pH 5,9 avant autoclavage

### DEV

2,2 g macro et micro-éléments de Murashige et Skoog (ref. M 6899 Sigma)
2 ml/l vitamines de Nitsch
15 g/l saccharose
50 mg/l Kanamycine
400 mg/l Augmentin
8 g/l Agar-Agar
pH 5,9 avant autoclavage

### MS20

4,4 g/l M0404 (SIGMA)
20 g/l Saccharose
8 g/l Agar-Agar (milieu solide)
pH 5,7
+ éventuellement hormones végétales BAP 1 mg/l et ANA 0,1 mg/l

### MS30

4,4 g/l M0404 (SIGMA)
30 g/l Saccharose
8 g/l Agar-Agar (milieu solide)
pH 5,7
+ éventuellement hormones végétales BAP 1 mg/l et ANA 0,1 mg/l

### BIBLIOGRAPHIE

- An G.(1986), Plant Physiol. 81 : 86-91
- Bednarek S.Y et Ralkhel N.V., The Plant Cell, 3, 1.195-1206 (1991)
- Broer et al., Braunschweig Symposium on Applied Plant Molecular biology, Braunschweig November 21-23, (1988) ; Proceedings,Ed. Galling G., (1989), 240
- Berg D.E., Berg C.M., (1983) Biotechnology, 1, 417-435
- Close J.J., Rodriguez R.L., (1982) Gene, 20, 305-316
- Cocherel S., Perez P., Degroote F., Genestier S., Picard G., (1996), Plant Molecular Biology 30 : 539-551
- De la Penna (1987), Nature, 325:274-278
- Depicker et al., (1982) J. Mol. Appl. Genet., 1, 561-573
- Ditta G., Stanfield S., Corbin D., Helinski D.R., (1980) Pnas 77 : 7347-7351
- Ellstrand Norman C., (1990), Bioscience, vol.40 n° 6, pp 438-442
- Fillatti J.J., Kiser J., Rose R. et Commaî L., (1987) Bio/Technology 5 : 726-730
- Finnegan E.J., Lawrence G.J., Dennis E.S., Ellis J.G., (1993) Plant Molecular Biology 22 : 625-633
- Franck et al., (1980) Cell. 21,285-294
- Fromm M.E., Taylor L.P., Walbot V., (1986) Nature, vol. 319, 791-793
- Gaubier et al., Mol. Gen., 238, 409-418 (1993)
- Holsters et al., (1978) Mol. Gen.,Genet., 163, 181-187
- Horsch et al., (1985), Science, 227, 1229 (1985)
- Jefferson R.A., (1987) Plant Molecular Biology, Rep. 5 : 387-405
- Jouanin, Plant. Sci., 53, 53-63 (1987)
- Kay, Science, (1987) 236, 1299-1302
- Lassner M.W., Peterson P., Yoder J.I.,(1989), Plant Molecular Biology Rep. 7: 116-128
- Liu, (1951) Mol. Plant. Microb., Interactions, 6, 144-156
- Matsuoka K., Nakamura K., (1991) Proc. Natl. Acad. Sci. USA, 88, 834-838
- Mc Elroy (1991) Mol. Gen. Genet. 231 : 150-160
- Murakami et al., Plant Mol. Biol., (1986) 7, 343-355
- Ni et al., Plnt J., (1995) 7, 661-676
- Paul W. et al. (1992) Plant. Mol. Biol. 19 : 611-622
- Perez P., Tiraby G., Kallerhoff J., Perret J., (1989), Plant Molecular Biology 13 : 365-373
- Rogers S., Bendich A.J., (1988), Plant Molecular Biology, Manual A65, 69-76
- Sambrook J., Fritsch E.F., Maniatis T., (1989) Molecular cloning, a laboratory manual, 2nd edition, Cold Spring Harbor laboratory press
- Sanford J.C., (1988) Trends in Biotechnology, 6, 299-302
- Schroeder M.R., Borkhsenious O.N, Matsuoka K., Nakamura K. et Rakhel N.V., (1993) Plant. Physiol., 101, 451-458
- Watson et al., ADN recombinant, Ed. De Boeck Université, p 273-292
- WeiderR., Koornnef M., Zabel P., (1989) Theo. Appl. Gen., 78 : 169-172
- Worall D. Hird D.L., Hodge R., Paul W., Draper J., and Scott R., (1992), Plant Cell 4, 759-771
- Yoder J.I. Goldsbrough A.P., (1994), Bio/Technology vol. 12, 263-267

## Revendications

1. Utilisation d'une plante mâle stérile artificielle choisie parmi le maïs, le colza ou la tomate, comprenant un gène de stérilité mâle artificielle (gène AMS), dans le génome de laquelle est intégré un transgène d'intérêt dont l'expression n'est pas empêchée par la présence du gène AMS, pour éviter la dissémination dudit transgène.

2. Utilisation selon la revendication 1 dans laquelle ladite plante est porteuse d'une stérilité mâle cytoplasmique.

3. Utilisation selon la revendication 1 dans laquelle ladite plante est porteuse d'une stérilité mâle nucléaire.

4. Utilisation selon l'une des revendications 1 à 3, **caractérisée en ce que** le transgène d'intérêt code pour une protéine d'intérêt thérapeutique ou prophylactique.

5. Utilisation selon la revendication 4, **caractérisée en ce que** le transgène d'intérêt est choisi parmi une protéine codant pour le collagène ou la lipase gastrique.

6. Plante transgénique, ou partie de plante transgénique, choisie parmi le maïs, le colza ou la tomate **caractérisée en ce** ladite plante transgénique ou ladite partie de plante transgénique comprend un transgène d'intérêt lié génétiquement à un gène de stérilité mâle artificielle (gène AMS) associé à des éléments permettant son expression dans les cellules végétales, l'expression dudit transgène n'étant pas empêchée par la présence du gène AMS.

7. Plante transgénique ou partie de plante transgénique selon la revendication 6, **caractérisée en ce que** les éléments permettant l'expression dans les cellules végétales sont un promoteur et un terminateur de transcription.

8. Plante transgénique ou partie de plante transgénique selon l'une des revendications 6 ou 7, **caractérisée en ce que** le gène de stérilité mâle artificielle (gène AMS) est associé à un promoteur permettant une expression spécifique dans l'anthère.

9. Plante transgénique ou partie de plante transgénique selon l'une quelconque des revendications 6 à 8, **caractérisée en ce que** transgène d'intérêt code pour une protéine d'intérêt thérapeutique ou prophylactique.

10. Plante transgénique ou partie de plante transgénique selon la revendication 9, **caractérisée en ce que** le transgène d'intérêt est choisi parmi une protéine codant pour le collagène ou la lipase gastrique.

11. Procédé de production d'un produit d'expression d'un transgène d'intérêt **caractérisé en ce qu'**il comprend :
a)
- soit la transformation de cellules végétales de maïs, de colza ou de tomate, à l'aide d'un hôte cellulaire transformé par un vecteur contenant à la fois un transgène d'intérêt associé à des éléments permettant son expression dans les cellules végétales, et un gène de stérilité mâle artificielle (gène AMS) lié génétiquement audit transgène d'intérêt, ledit gène AMS étant associé à des éléments permettant son expression dans les cellules végétales, la présence dudit gène AMS n'empêchant pas l'expression dudit transgène d'intérêt, de manière à intégrer dans le génome de ces cellules un gène AMS lié génétiquement à un transgène d'intérêt ;
- soit la transformation de cellules végétales de maïs, de colza ou de tomate porteuses d'une stérilité mâle cytoplasmique ou nucléaire de manière à intégrer un transgène d'intérêt dans le génome de ces cellules;
b) la régénération à partir des cellules végétales transformées susmentionnées, de plantes transformées présentant un phénotype mâle stérile et exprimant ledit transgène d'intérêt ;
c) la récupération du produit d'expression dudit transgène d'intérêt dans lesdites plantes transformées sus-mentionnées.

12. Procédé de production d'un produit d'expression d'un transgène d'intérêt selon la revendication 11, **caractérisée en ce que** la récupération du produit d'expression dudit transgène dans l'étape c) est réalisée par extraction.

13. Procédé de production d'un produit d'expression d'un transgène d'intérêt selon l'une des revendications 11 ou 12, **caractérisé en ce que** la récupération du produit d'expression dudit transgène dans l'étape c) est suivie par une purification.

14. Procédé de production d'un produit d'expression d'un transgène d'intérêt selon l'une quelconque des revendications 11 à 13, **caractérisé en ce que** ledit transgène d'intérêt code pour une protéine d'intérêt thérapeutique ou prophylactique.

15. Procédé de production d'un produit d'expression d'un transgène d'intérêt selon la revendication 14, **caractérisé en ce que** le transgène d'intérêt est choisi parmi une protéine codant pour le collagène ou la lipase gastrique.

## Claims

1. Use of a male, sterile, artificial plant chosen from among maize, colza or tomato, comprising an artificial male sterility gene (AMS gene), in the genome of which is integrated a transgene which is of interest and whose expression is not prevented by the presence of the AMS gene, in order to avoid the dissemination of said transgene.

2. Use according to claim 1, wherein said plant is the carrier of a cytoplasmic male sterility.

3. Use according to claim 1, wherein said plant is the carrier of a nuclear male sterility.

4. Use according to one of the claims 1 to 3, **characterized in that** the transgene of interest codes for a protein of therapeutic or prophylactic interest.

5. Use according to claim 4, **characterized in that** the transgene of interest is chosen from among a protein coding for collagen or gastric lipase.

6. Transgenic plant, or transgenic plant part, chosen from among maize, colza or tomato, **characterized in that** said transgenic plant or said transgenic plant part comprising a transgene of interest bound genetically to an artificial male sterility gene (AMS gene) associated with elements permitting its expression in plant cells, the expression of said transgene not being prevented by the presence of the AMS gene.

7. Transgenic plant or transgenic plant part according to claim 6, **characterized in that** the elements permitting the expression in the plant cells are a transcription promoter and terminator.

8. Transgenic plant or transgenic plant part according to one of the claims 6 or 7, **characterized in that** the artificial male sterility gene (AMS gene) is associated with a promoter permitting a specific expression in the anther.

9. Transgenic plant or transgenic plant part according to any one of the claims 6 to 8, **characterized in that** the transgene of interest codes for a protein of therapeutic or prophylactic interest.

10. Transgenic plant or transgenic plant part according to claim 9, **characterized in that** the transgene of interest is chosen from among a protein coding for collagen or gastric lipase.

11. Process for the production of an expression product of a transgene of interest, **characterized in that** it comprises:
a) either the transformation of plant cells of maize, colza or tomato, with the aid of a cellular host transformed by a vector containing both a transgene of interest associated with elements permitting its expression in plant cells, and an artificial male sterility gene (AMS gene) bound genetically to said transgene of interest, said AMS gene being associated with elements permitting its expression in plant cells, the presence of said AMS gene not preventing the expression of said transgene of interest, so as to integrate into the genome of said cells an AMS gene genetically bound to a transgene of interest,
- or the transformation of plant cells of maize, colza or tomato carrying a male cytoplasmic or nuclear sterility so as to integrate a transgene of interest into the genome of said cells,
b) the regeneration from the aforementioned, transformed plant cells of transformed plants having a male, sterile phenotype and expressing said transgene of interest,
c) the recovery of the expression product of said transgene of interest in said aforementioned transformed plants.

12. Process for the production of an expression product of a transgene of interest according to claim 11, **characterized in that** the recovery of the expression product of said transgene in stage c) is implemented by extraction.

13. Process for the production of an expression product of a transgene of interest according to one of the claims 11 or 12, **characterized in that** the recovery of the expression product of said transgene in stage c) is followed by a purification.

14. Process for the production of an expression product of a transgene of interest according to any one of the claims 11 to 13, **characterized in that** said transgene of interest codes for a protein of therapeutic or prophylactic interest.

15. Process for the production of an expression product of a transgene of interest according to claim 14, **characterized in that** the transgene of interest is chosen from among a protein coding for collagen or gastric lipase.

## Patentansprüche

1. Verwendung einer künstlich männlich sterilen Pflanze, die aus Mais, Hirse oder Tomate ausgewählt ist, ein künstliches männliches Sterilitätsgen (AMS-Gen) enthält und in deren Genom ein interessierendes Transgen integriert ist, dessen Expression vom Vorhandensein des AMS-Gens nicht behindert wird, um die Verbreitung des Transgens zu verhindern.

2. Verwendung nach Anspruch 1, in welcher die Pflanze eine cytoplasmatische männliche Sterilität trägt.

3. Verwendung nach Anspruch 1, in welcher die Pflanze eine nucleäre männliche Sterilität trägt.

4. Verwendung nach einem der Ansprüche 1 bis 3, **dadurch gekennzeichnet, dass** das interessierende Transgen für ein interessierendes therapeutisches oder prophylaktisches Protein codiert.

5. Verwendung nach Anspruch 4, **dadurch gekennzeichnet, dass** das interessierende Transgen aus einem Protein ausgewählt ist, das für Collagen oder die gastrische Lipase codiert.

6. Transgene Pflanze oder transgener Teil einer Pflanze, die aus Mais, Raps oder Tomate ausgewählt ist, **dadurch gekennzeichnet, dass** die transgene Pflanze oder der transgene Pflanzenteil ein interessierendes Transgen enthält, das genetisch mit einem künstlichen männlichen Sterilitätsgen (AMS-Gen) verknüpft ist, das mit Elementen verbunden ist, welche seine Expression in Pflanzenzellen erlauben, wobei die Expression des Transgens nicht von dem Vorhandensein des AMS-Gens behindert wird.

7. Transgene Pflanze oder transgener Pflanzenteil nach Anspruch 6, **dadurch gekennzeichnet, dass** die Elemente, welche die Expression in den Pflanzenzellen erlauben, ein Transkriptionspromotor und ein Transkriptionsterminator sind.

8. Transgene Pflanze oder transgener Pflanzenteil nach Anspruch 6 oder 7, **dadurch gekennzeichnet, dass** das künstliche männliche Sterilitätsgen (AMS-Gen) mit einem Promotor verknüpft ist, der eine spezifische Expression im Staubbeutel erlaubt.

9. Transgene Pflanze oder transgener Pflanzenteil nach einem der Ansprüche 6 bis 8, **dadurch gekennzeichnet, dass** das interessierende Transgen für ein interessierendes therapeutisches oder prophylaktisches Protein codiert.

10. Transgene Pflanze oder transgener Pflanzenteil nach Anspruch 9, **dadurch gekennzeichnet, dass** das interessierende Transgen aus einem Protein ausgewählt ist, das für Collagen oder die gastrische Lipase codiert.

11. Verfahren zur Herstellung eines Expressionsprodukts eines interessierenden Transgens, **dadurch gekennzeichnet, dass** es:
a)
- entweder die Transformation von Mais-, Raps- oder Tomatenpflanzenzellen mittels eines Zellwirts, der von einem Vektor transformiert ist, der gleichzeitig ein interessierendes Transgen, das mit Elementen verbunden ist, die seine Expression in den Pflanzenzellen erlauben, und ein künstliches männliches Steriliätsgen (AMS-Gen), das genetisch mit dem interessierenden Transgen verknüpft ist, enthält, wobei das AMS-Gen mit Elementen verbunden ist, die dessen Expression in den Pflanzenzellen erlauben, und das Vorhandensein dieses AMS-Gens die Expression des interessierenden Transgens nicht behindert, sodass in das Genom dieser Zellen ein AMS-Gen integriert wird, das genetisch mit einem interessierenden Transgen verknüpft ist, oder
- die Transformation von Mais-, Raps- oder Tomatenpflänzenzellen, die eine cytoplasmatische oder nucleäre männliche Sterilität, derart tragen, dass ein interessierendes Transgen in das Genom dieser Zellen integriert wird,
a) die Regeneration ausgehend von diesen transformierten Pflanzenzellen zu transformierten Pflanzen, die einen männlich sterilen Phänotyp aufweisen und das interessierende Transgen exprimieren, und
b) die Gewinnung des Expressionsproduktes des interessierenden Transgens in diesen transformierten Pflanzen umfasst.

12. Verfahren zur Herstellung eines Expressionsproduktes eines interessierenden Transgens nach Anspruch 11, **dadurch gekennzeichnet, dass** die Gewinnung des Expressionsproduktes des Transgens in Stufe c) durch Extraktion erfolgt.

13. Verfahren zur Herstellung eines Expressionsproduktes eines interessierenden Transgens nach Anspruch 11 oder 12, **dadurch gekennzeichnet, dass** auf die Gewinnung des Expressionsproduktes des Transgens in Stufe c) eine Reinigung folgt.

14. Verfahren zur Herstellung eines Expressionsproduktes eines interessierenden Transgens nach einem der Ansprüche 11 bis 13, **dadurch gekennzeichnet, dass** das interessierende Transgen für ein interessierendes therapeutisches oder prophylaktisches Protein codiert.

15. Verfahren zur Herstellung eines Expressionsproduktes eines interessierenden Transgens nach Anspruch 14, **dadurch gekennzeichnet, dass** das interessierende Transgen aus einem für Collagen oder die gastrische Lipase codierenden Protein ausgewählt ist.
